# EUROPEAN PATENT APPLICATION

(11) **EP 4 799 655 A1**
(43) Date of publication of application: **02.09.2026**
(21) Application number: 24882398.1
(22) Date of filing: 22.10.2024
(51) Int. Cl.: A61L 2/20, A61L 101/22

(54) **METHOD FOR REMOVING HYDROGEN PEROXIDE**

(30) Priority: 23.10.2023 JP 2023182110; 31.10.2023 JP 2023186270
(71) Applicant: MIURA CO., LTD., Matsuyama-shi Ehime 799-2696 (JP); Institute of Science Tokyo, Tokyo 152-8550 (JP); Central Institute for Experimental Medicine and Life Science, Kawasaki-shi, Kanagawa 210-0821 (JP)
(72) Inventor: HAISHIMA, Yuji, Matsuyama-shi, Ehime 799-2696 (JP); NAKAMURA, Yasuo, Matsuyama-shi, Ehime 799-2696 (JP); FUKUZUMI, Masahiro, Matsuyama-shi, Ehime 799-2696 (JP); MIZOBE, Tomoyuki, Matsuyama-shi, Ehime 799-2696 (JP); TAGAWA, Yoh-ichi, Tokyo 152-8550 (JP); CHEN, Zhiping, Tokyo 152-8550 (JP); TAMURA, Shiori, Tokyo 152-8550 (JP); SASAKI, Erika, Kawasaki-shi, Kanagawa 210-0821 (JP); SATO, Kenya, Kawasaki-shi, Kanagawa 210-0821 (JP); SHINOHARA, Haruka, Kawasaki-shi, Kanagawa 210-0821 (JP)
(74) Representative: SSM Sandmair
(86) International application number: PCT/JP2024/037638
(87) International publication number: WO 2025/089287

(57) **Abstract**

A method for removing hydrogen peroxide remaining in an object includes a depressurization step (SB15) of depressurizing an internal space of a chamber (11) accommodating the object, a water vapor supply step (SB16) of supplying water vapor to the internal space in a state where the internal space is depressurized, and an atmospheric air supply step (SB17) of supplying atmospheric air to the internal space after the water vapor is supplied to the internal space.

## Description

### TECHNICAL FIELD

The present application claims priority based on Japanese Patent Application No. 2023-182110 filed in Japan on October 23, 2023, and Japanese Patent Application No. 2023-186270 filed in Japan on October 31, 2023, the contents of which are incorporated herein by reference.

The technology disclosed in the present specification relates to a method for removing hydrogen peroxide.

### BACKGROUND ART

In the technical field related to a sterilization device, a sterilization device using hydrogen peroxide as disclosed in Patent Literature 1 is known.

### PRIOR ART LITERATURE

### PATENT LITERATURE

Patent Literature 1: WO 2020/183696 A

### DISCLOSURE OF THE INVENTION

### PROBLEM TO BE SOLVED BY THE INVENTION

There is a demand for a technology for sterilizing an object using hydrogen peroxide and then removing the hydrogen peroxide remaining in the object.

The technology disclosed in the present specification aims to remove hydrogen peroxide remaining in an object.

### SOLUTION TO THE PROBLEM

The present specification discloses a method for removing hydrogen peroxide remaining in an object. A method for removing hydrogen peroxide includes a depressurization step of depressurizing an internal space of a chamber accommodating an object, a water vapor supply step of supplying water vapor to the internal space in a state where the internal space is depressurized, and an atmospheric air supply step of supplying atmospheric air to the internal space after the water vapor is supplied to the internal space.

### EFFECT OF THE INVENTION

According to the technology disclosed in the present specification, hydrogen peroxide remaining in an object is removed.

### BRIEF DESCRIPTION OF THE DRAWINGS

Fig. 1 is a diagram schematically illustrating a sterilization device according to a first embodiment.
Fig. 2 is a flowchart illustrating an operation of the sterilization device according to the first embodiment.
Fig. 3 is a flowchart illustrating removal processing according to the first embodiment.
Fig. 4 is a diagram showing a variation in the amount of hydrogen peroxide remaining on a 96 well plate in a depressurization/drying step.
Fig. 5 is a diagram for describing an effect of the removal processing according to the first embodiment.
Fig. 6 is a flowchart illustrating removal processing according to a second embodiment.

### DESCRIPTION OF PREFERRED EMBODIMENTS

Hereinafter, embodiments according to the present disclosure will be described with reference to the drawings, but the present disclosure is not limited to the embodiments. The components of the embodiments described below can be appropriately combined. In addition, some components may be omitted.

### [First embodiment]

A first embodiment will be described.

### <Sterilization device>

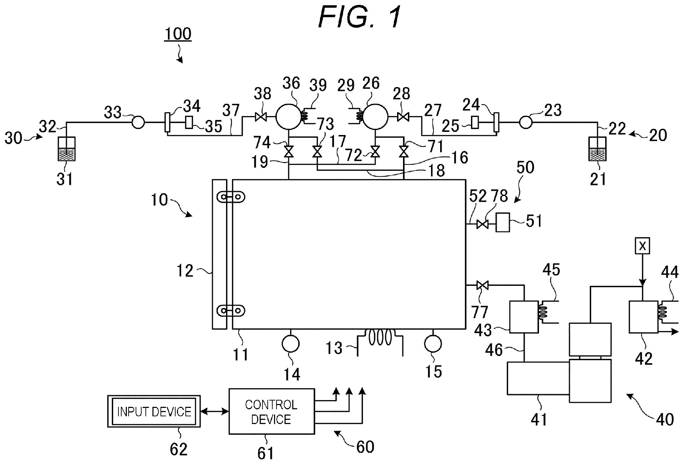

Fig. 1 is a diagram schematically illustrating a sterilization device 100 according to the present embodiment. The sterilization device 100 sterilizes an object using hydrogen peroxide gas (H₂O₂). Examples of the object include parts used for cell culture. Examples of the parts include a petri dish and a well plate. Note that the object may be a medical instrument. Examples of the medical instrument include steel products such as forceps, tweezers, and scissors, and resin products such as tubes.

The sterilization device 100 includes a chamber unit 10, a hydrogen peroxide supply unit 20, a vapor cleaning unit 30, a depressurization unit 40, a repressurization unit 50, and a control unit 60.

The chamber unit 10 includes a chamber 11 including a door 12, a heating unit 13, a pressure sensor 14, and a temperature sensor 15.

The chamber 11 accommodates an object. The chamber 11 has an internal space in which an object is disposed. The door 12 opens and closes an opening provided in the chamber 11. The object is carried into the internal space of the chamber 11 through the opening of the chamber 11. When the door 12 is closed, the internal space of the chamber 11 is sealed.

The heating unit 13 is connected to the chamber 11. The heating unit 13 adjusts the temperature of the internal space of the chamber 11.

The pressure sensor 14 is connected to the chamber 11. The pressure sensor 14 detects the pressure in the internal space of the chamber 11.

The temperature sensor 15 is connected to the chamber 11. The temperature sensor 15 detects the temperature of the internal space of the chamber 11.

The hydrogen peroxide supply unit 20 supplies hydrogen peroxide gas to the internal space of the chamber 11 in sterilization processing of the object. The hydrogen peroxide supply unit 20 includes a bottle 21, an extraction line 22, a tube pump 23, a reservoir 24, a filter 25, an evaporator 26, a supply line 27, a valve 28, and a heater 29.

The bottle 21 accommodates an aqueous solution of hydrogen peroxide.

The extraction line 22 connects the bottle 21 and the reservoir 24. The aqueous solution of hydrogen peroxide extracted from the bottle 21 is supplied to the reservoir 24 via the extraction line 22.

The tube pump 23 is disposed in the extraction line 22. The tube pump 23 operates such that the aqueous solution of hydrogen peroxide is supplied from the bottle 21 to the reservoir 24.

The reservoir 24 temporarily stores a prescribed amount of aqueous solution of hydrogen peroxide extracted from the bottle 21. Air (atmospheric air) around the reservoir 24 is introduced into the reservoir 24 via the filter 25. The pressure in the reservoir 24 is atmospheric pressure. A HEPA filter is exemplified as the filter 25.

The evaporator 26 is connected to the reservoir 24 via the supply line 27. The evaporator 26 evaporates the aqueous solution of hydrogen peroxide supplied from the reservoir 24 to generate hydrogen peroxide gas.

The valve 28 is disposed in the supply line 27. An electromagnetic valve is exemplified as the valve 28. When the valve 28 is opened, the aqueous solution of hydrogen peroxide in the reservoir 24 is sucked into the depressurized evaporator 26. As described above, air (atmospheric air) around the reservoir 24 is introduced into the reservoir 24 via the filter 25. When the valve 28 is opened, air (atmospheric air) is sucked into the evaporator 26 together with the aqueous solution of hydrogen peroxide.

The evaporator 26 is connected to the chamber 11 via an injection line 16 and an injection line 17. A valve 71 is disposed in the injection line 16, and a valve 72 is disposed in the injection line 17. An electromagnetic valve is exemplified as the valve 71 and the valve 72. The hydrogen peroxide gas is generated in the evaporator 26, and the valve 71 or the valve 72 is opened in a state where the pressure of the evaporator 26 increases, whereby the hydrogen peroxide gas is supplied to the internal space of the chamber 11.

The heater 29 adjusts the temperature of the evaporator 26. The heater 29 maintains the evaporator 26 at a predetermined temperature.

The vapor cleaning unit 30 supplies water vapor to the internal space of the chamber 11 in hydrogen peroxide removal processing performed after the sterilization processing. The vapor cleaning unit 30 includes a bottle 31, an extraction line 32, a tube pump 33, a reservoir 34, a filter 35, an evaporator 36, a supply line 37, a valve 38, and a heater 39.

The bottle 31 accommodates water.

The extraction line 32 connects the bottle 31 and the reservoir 34. The water extracted from the bottle 31 is supplied to the reservoir 34 via the extraction line 32.

The tube pump 33 is disposed in the extraction line 32. The tube pump 33 operates such that water is supplied from the bottle 31 to the reservoir 34.

The reservoir 34 temporarily stores a prescribed amount of water extracted from the bottle 31. Air (atmospheric air) around the reservoir 34 is introduced into the reservoir 34 via the filter 35. The pressure in the reservoir 34 is atmospheric pressure. A HEPA filter is exemplified as the filter 35.

The evaporator 36 is connected to the reservoir 34 via the supply line 37. The evaporator 36 evaporates water supplied from the reservoir 34 to generate water vapor.

The valve 38 is disposed in the supply line 37. An electromagnetic valve is exemplified as the valve 38. When the valve 38 is opened, the water in the reservoir 34 is sucked into the depressurized evaporator 36. As described above, air (atmospheric air) around the reservoir 34 is introduced into the reservoir 34 via the filter 35. When the valve 38 is opened, air (atmospheric air) is sucked into the evaporator 36 together with water.

The evaporator 36 is connected to the chamber 11 via an injection line 18 and an injection line 19. A valve 73 is disposed in the injection line 18, and a valve 74 is disposed in the injection line 19. An electromagnetic valve is exemplified as the valve 73 and the valve 74. Water vapor is generated in the evaporator 36, and the valve 73 or the valve 74 is opened in a state where the pressure of the evaporator 36 increases, whereby the water vapor is supplied to the internal space of the chamber 11.

The heater 39 adjusts the temperature of the evaporator 36. The heater 39 maintains the evaporator 36 at a predetermined temperature.

The depressurization unit 40 discharges gas from the internal space of the chamber 11. The depressurization unit 40 depressurizes the internal space of the chamber 11 by discharging gas from the internal space of the chamber 11. The depressurization unit 40 includes a vacuum pump 41, a first catalyst tank 42, a second catalyst tank 43, a first heater 44, and a second heater 45.

The vacuum pump 41 is connected to the chamber 11 via an exhaust line 46. The vacuum pump 41 operates so that gas is discharged from the internal space of the chamber 11. A valve 77 is disposed in the exhaust line 46. In the case of depressurizing the internal space of the chamber 11, when the pressure in the internal space of the chamber 11 reaches a default value, the valve 77 is closed and the vacuum pump 41 is stopped.

The first catalyst tank 42 is disposed on the upstream side of the vacuum pump 41. The second catalyst tank 43 is disposed on the downstream side of the vacuum pump 41. The catalyst decomposes hydrogen peroxide using, for example, manganese dioxide as a main component.

The first heater 44 keeps the first catalyst tank 42 warm. The second heater 45 keeps the second catalyst tank 43 warm.

The repressurization unit 50 repressurizes the internal space of the chamber 11 depressurized by the depressurization unit 40. The repressurization unit 50 introduces air (atmospheric air) in the external space of the chamber 11 into the internal space of the chamber 11 to repressurize the internal space of the chamber 11. The repressurization unit 50 includes a filter 51 and an introduction line 52.

The filter 51 collects foreign matter from air (atmospheric air) introduced into the internal space of the chamber 11. A HEPA filter is exemplified as the filter 51.

The introduction line 52 connects the internal space and the external space of the chamber 11. Air (atmospheric air) introduced from the external space of the chamber 11 into the internal space of the chamber 11 flows through the introduction line 52. Air (atmospheric air) in the external space of the chamber 11 is introduced into the internal space of the chamber 11 via the filter 51.

A valve 78 is disposed in the introduction line 52. An electromagnetic valve is exemplified as the valve 78. The valve 78 adjusts the flow rate of air in the introduction line 52. When the valve 78 is opened, air (atmospheric air) in the external space of the chamber 11 is introduced into the internal space of the chamber 11 via the introduction line 52. When the valve 78 is closed, the introduction of air (atmospheric air) into the internal space of the chamber 11 is stopped.

The control unit 60 controls the components of the sterilization device 100. The control unit 60 includes a control device 61 and an input device 62. The control device 61 includes a computer system. Examples of the input device 62 include a touch panel and a computer keyboard. The control device 61 can control components of the sterilization device 100, for example, on the basis of input data input via the input device 62.

### <Operation of sterilization device>

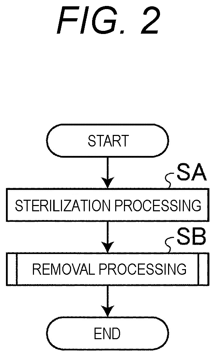

Fig. 2 is a flowchart illustrating an operation of the sterilization device 100 according to the present embodiment. In the present embodiment, the sterilization device 100 performs the sterilization processing SA and removal processing SB. The removal processing SB is performed after the sterilization processing SA. The removal processing SB is processing of removing hydrogen peroxide remaining in the object after the sterilization processing SB.

### (Sterilization processing)

When an object is subjected to sterilization processing, the object is accommodated in the chamber 11. The user of the sterilization device 100 carries the object into the internal space of the chamber 11 through the opening of the chamber 11. After the object is placed in the internal space of the chamber 11, the user closes the door 12. When the door 12 is closed, the internal space of the chamber 11 is sealed.

After the internal space of the chamber 11 is sealed, the control device 61 controls the depressurization unit 40 so that the gas is discharged from the internal space of the chamber 11. After the gas is discharged from the internal space of the chamber 11, the control device 61 controls the hydrogen peroxide supply unit 20 so that the hydrogen peroxide gas is supplied to the internal space of the chamber 11. The control device 61 controls the heating unit 13 so that the internal space of the chamber 11 is heated to a predetermined sterilization temperature. The hydrogen peroxide gas is supplied to the internal space of the chamber 11 in a state where the internal space of the chamber 11 is heated to the sterilization temperature. As an example, the sterilization temperature is approximately 50°C. When the hydrogen peroxide gas is supplied to the internal space of the chamber 11, the hydrogen peroxide gas comes into contact with the surface of the object, and the object is sterilized with hydrogen peroxide.

### (Removal processing)

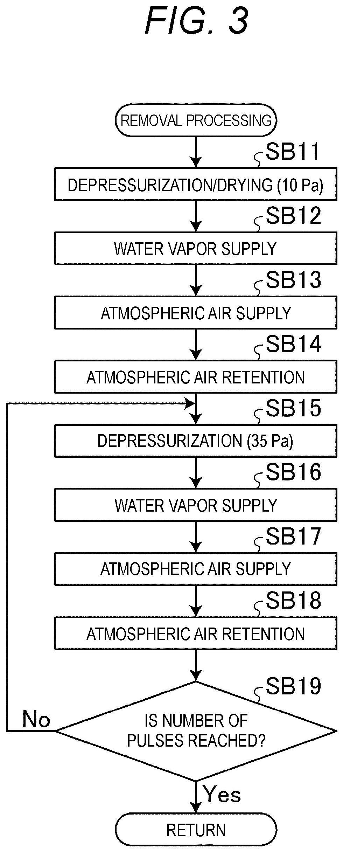

After the sterilization processing is completed, the removal processing SB for removing hydrogen peroxide remaining in the object is performed. Fig. 3 is a flowchart illustrating the removal processing SB according to the present embodiment. In the present embodiment, the removal processing SB includes a depressurization/drying step SB11, a water vapor supply step SB12, an atmospheric air supply step SB13, an atmospheric air retention step SB14, a depressurization step SB15, a water vapor supply step SB16, an atmospheric air supply step SB17, and an atmospheric air retention step SB18.

After the sterilization processing is completed, the depressurization/drying step SB11 is performed in a state where the object is accommodated in the chamber 11. In the depressurization/drying step SB11, the control device 61 controls the depressurization unit 40 so that the internal space of the chamber 11 accommodating the object is depressurized. In the depressurization/drying step SB11, the control device 61 depressurizes the internal space of the chamber 11 so that the pressure of the internal space of the chamber 11 reaches approximately 10 Pa (second pressure). In the depressurization/drying step SB11, the control device 61 controls the heating unit 13 so that the temperature of the internal space of the chamber 11 reaches a depressurization/drying temperature higher than the sterilization temperature. As an example, the depressurization/drying temperature is approximately 80°C.

In the depressurization/drying step SB11, the pressure in the internal space of the chamber 11 is adjusted to approximately 10 Pa, and the state where the temperature is adjusted to approximately 80°C is maintained for approximately 24 hours (second time).

In the depressurization/drying step SB11, when the internal space of the chamber 11 is depressurized, hydrogen peroxide remaining in the object is efficiently evaporated, so that hydrogen peroxide is removed from the object. Note, however, that when a certain period of time elapses from the start time of the depressurization/drying step SB11, the evaporation amount of hydrogen peroxide decreases.

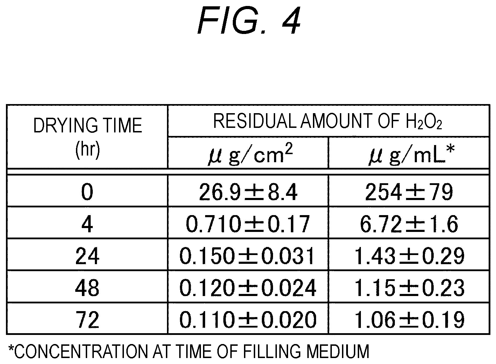

Fig. 4 is a diagram showing a variation in the amount of hydrogen peroxide remaining on a 96 well plate in the depressurization/drying step SB11. As shown in Fig. 4, the hydrogen peroxide remaining on the sterilized 96 well plate was reduced in a quadratic curve by the depressurization/drying step, but it was found that the removal effect was limited to a concentration of about 1.0 µg/mL at the time of filling the medium. Therefore, in the present embodiment, the control device 61 ends the depressurization/drying step SB11 when approximately 24 hours have elapsed from the start time of the depressurization/drying step SB11. Note that as a cause of the decrease in the evaporation amount of hydrogen peroxide, the concentration of hydrogen peroxide remaining in the object is high, or it is difficult to evaporate due to bonding of hydrogen peroxide to the object.

After completion of the depressurization/drying step SB11, the water vapor supply step SB12 is performed. In the water vapor supply step SB12, the control device 61 controls the vapor cleaning unit 30 so that water vapor is supplied to the internal space of the chamber 11 in a state where the internal space of the chamber 11 is depressurized to approximately 10 Pa.

In the water vapor supply step SB12, water vapor is supplied to the internal space of the chamber 11.

Note that the amount of water vapor supplied to the internal space of the chamber 11 is small. As an example, the amount of water vapor supplied to the internal space of the chamber 11 is approximately 10cc. When a large amount of water vapor is supplied to the internal space of the chamber 11, there is a possibility that the vacuum pump 41 of the depressurization unit 40 sucks a large amount of water vapor in the depressurization step SB15 performed after the water vapor supply step SB12. In the present embodiment, the amount of water vapor supplied to the internal space of the chamber 11 is determined in advance. The amount of water vapor supplied to the internal space of the chamber 11 is determined so as not to affect the vacuum pump 41 and to be able to remove hydrogen peroxide from the object.

Note that when a small amount of water vapor is supplied to the internal space of the chamber 11, the pressure in the internal space of the chamber 11 increases to approximately 20 Pa.

After the water vapor supply step SB12 is completed, the atmospheric air supply step SB13 is performed. In the atmospheric air supply step SB13, the control device 61 controls the repressurization unit 50 so that the atmospheric air is supplied to the internal space of the chamber 11. As described above, the repressurization unit 50 has the filter 51 such as a HEPA filter. Clean air (atmospheric air) that has passed through the filter 51 is supplied to the internal space of the chamber 11. When air (atmospheric air) is supplied to the internal space of the chamber 11, the pressure in the internal space of the chamber 11 increases. The control device 61 may increase the pressure in the internal space of the chamber 11 to the atmospheric pressure or to a pressure lower than the atmospheric pressure.

In the present embodiment, in the atmospheric air supply step SB13, since the pressure in the internal space of the chamber 11 increases to a pressure (approximately 90000 Pa) lower than the atmospheric pressure, the supplied water vapor is diffused over the entire surface of the object.

After the atmospheric air supply step SB13 is completed, the atmospheric air retention step SB14 of maintaining the pressure in the internal space of the chamber 11 at approximately 90000 Pa is performed. As a result, hydrogen peroxide remaining in the object is dissolved in moisture (supplied water vapor) adhering to the entire surface of the object.

After the atmospheric air retention step SB14 is completed, the depressurization step SB15 is performed. In the depressurization step SB15, the control device 61 controls the depressurization unit 40 so that the internal space of the chamber 11 accommodating the object is depressurized. In the depressurization step SB15, the control device 61 depressurizes the internal space of the chamber 11 so that the pressure in the internal space of the chamber 11 reaches approximately 35 Pa (first pressure). In the depressurization step SB15, the control device 61 controls the heating unit 13 so as to be equal to the temperature of the internal space of the chamber 11. As an example, the depressurization temperature is approximately 50°C. As the chamber 11 is depressurized, hydrogen peroxide remaining on the surface of the object is removed together with moisture and discharged to the external space.

In the depressurization step SB15, the pressure in the internal space of the chamber 11 is adjusted to approximately 35 Pa, and the state where the temperature is adjusted to approximately 50°C is maintained for a time (first time) of one minute or more and 10 minutes or less. As an example, it is assumed that a state where the pressure in the internal space of the chamber 11 is adjusted to approximately 35 Pa and the temperature is adjusted to approximately 50°C is continued for approximately three minutes.

After the depressurization step SB15 is completed, the water vapor supply step SB16 is performed. The water vapor supply step SB16 is similar to the water vapor supply step SB12 described above.

After the water vapor supply step SB16 is completed, the atmospheric air supply step SB17 and the atmospheric air retention step SB18 are performed. The atmospheric air supply step SB17 is similar to the atmospheric air supply step SB13 described above, and the atmospheric air retention step SB18 is similar to the atmospheric air retention step SB14 described above.

A pulse step including the depressurization step SB15, the water vapor supply step SB16, the atmospheric air supply step SB17, and the atmospheric air retention step SB18 is performed a plurality of times. In the following description, the number of times of performing the pulse step including the depressurization step SB15, the water vapor supply step SB16, the atmospheric air supply step SB17, and the atmospheric air retention step SB18 is appropriately referred to as the number of pulses.

The number of pulses is a predetermined value. Note that the user may operate the input device 62 to input the number of pulses to the control device 61. The number of pulses may be arbitrarily determined by the user. The control device 61 determines whether or not the number of times the pulse step has been performed has reached the number of pulses (step SB19).

In step SB19, if it is determined that the number of pulses has not been reached (step SB19: No), the control device 61 repeats the processing from step SB15 to step SB18 until the number of pulse steps reaches the number of pulses.

In step SB19, if it is determined that the number of pulses has been reached (step SB19: Yes), the control device 61 ends the removal processing SB.

### <Effect>

As described above, in the present embodiment, the removal processing SB of hydrogen peroxide remaining in the object includes the depressurization step SB15 of depressurizing the internal space of the chamber 11 accommodating the object, the water vapor supply step SB16 of supplying water vapor to the internal space of the chamber 11 in a state where the internal space of the chamber 11 is depressurized, and the atmospheric air supply step SB17 of supplying atmospheric air to the internal space of the chamber 11 after water vapor is supplied to the internal space of the chamber 11.

According to the embodiment, since the water vapor supply step SB16 is performed, the hydrogen peroxide remaining in the object is dissolved in the water vapor and peeled off from the object. As a result, hydrogen peroxide remaining in the object is sufficiently removed.

For example, in the field of regenerative medicine, cell culture is performed in a cell culture isolator. In cell culture, parts such as petri dishes and well plates are used. The parts used for cell culture are used in the cell culture isolator after being sterilized in the sterilization device 100. When hydrogen peroxide remains in the part, cell culture is not properly performed. According to the present embodiment, the hydrogen peroxide residue on the object is reduced to less than 0.5 µg/mL as the concentration at the time of filling the medium, and the cell culture can be properly performed.

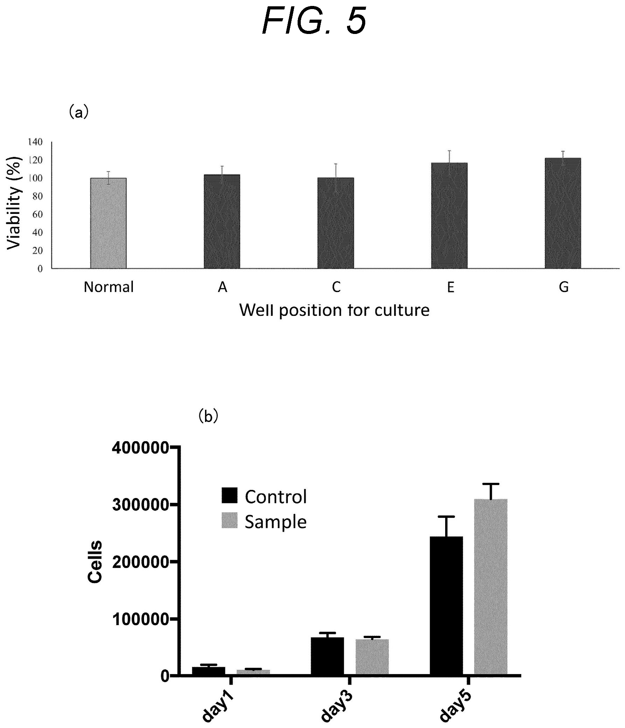

Fig. 5 is a diagram for describing an effect of the removal processing according to the present embodiment. Fig. 5a is a diagram showing the survival rate after 7 days when human iPS cells are cultured on a 96 well plate subjected to removal processing after sterilization processing according to the present embodiment. In Fig. 5a, Nomal is the negative control and A, C, E, and G represent plate rows. As shown in Fig. 5a, the influence of residual hydrogen peroxide was not observed, including rows A and G on the outer side of the plate where hydrogen peroxide easily remains, and the survival rate was equivalent to that of the negative control group. Fig. 5b is a diagram showing the cell numbers one day, three days, and five days after culturing the Marmoset ES cells on a 6 well plate. In Fig. 5b, Control is the negative control, and Sample represents a test group. As shown in Fig. 5b, no significant difference was observed between the negative control and the test group regardless of the culture period, and no influence of residual hydrogen peroxide was observed.

In the present embodiment, the pulse step including the depressurization step SB15, the water vapor supply step SB16, and the atmospheric air supply step SB17 is performed a plurality of times. By performing the pulse step a plurality of times, hydrogen peroxide remaining in the object is sufficiently removed.

In the present embodiment, the internal space of the chamber 11 reaches the first pressure (approximately 35 Pa) by the depressurization step SB15. Before the pulse step (depressurization step SB15), the depressurization/drying step SB11 of depressurizing the internal space of the chamber 11 to the second pressure (approximately 10 Pa) lower than the first pressure is performed. As a result, since hydrogen peroxide remaining in the object is efficiently evaporated, hydrogen peroxide is removed from the object.

In the present embodiment, in the depressurization step SB15, a state where the internal space of the chamber 11 is at the first pressure (approximately 35 Pa) is maintained for a first time (e.g., three minutes). In the depressurization/drying step SB11, a state where the internal space of the chamber 11 is at the second pressure (approximately 10 Pa) is maintained for a second time (e.g., 24 hours) longer than the first time. As a result, since the hydrogen peroxide remaining in the object is efficiently evaporated in the depressurization/drying step SB11, the hydrogen peroxide is removed from the object.

In the present embodiment, before the depressurization/drying step SB11, a sterilization step (sterilization processing SA) of sterilizing the object with hydrogen peroxide in the internal space of the chamber 11 is performed. As a result, the sterilization processing SA and the removal processing SB are continuously performed by one sterilization device 100.

### [Second embodiment]

A second embodiment will be described. In the following description, the same or equivalent components as those of the above-described embodiment are denoted by the same reference numerals, and the description of the components is simplified or omitted.

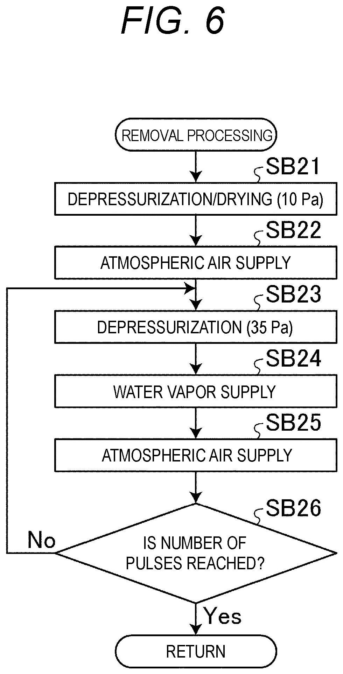

Fig. 6 is a flowchart illustrating removal processing SB according to the present embodiment. In the present embodiment, the removal processing SB includes a depressurization/drying step SB21, an atmospheric air supply step SB22, a depressurization step SB23, a water vapor supply step SB24, and an atmospheric air supply step SB25.

After sterilization processing is completed, the depressurization/drying step SB21 is performed in a state where an object is accommodated in a chamber 11. In the depressurization/drying step SB21, a control device 61 controls a depressurization unit 40 so that an internal space of the chamber 11 accommodating the object is depressurized. In the depressurization/drying step SB21, the control device 61 depressurizes the internal space of the chamber 11 so that the pressure of the internal space of the chamber 11 reaches approximately 10 Pa (second pressure). In the depressurization/drying step SB21, the control device 61 controls a heating unit 13 so that the temperature of the internal space of the chamber 11 reaches a depressurization/drying temperature higher than the sterilization temperature. As an example, the depressurization/drying temperature is approximately 80°C.

In the depressurization/drying step SB21, the pressure in the internal space of the chamber 11 is adjusted to approximately 10 Pa, and the state where the temperature is adjusted to approximately 80°C is maintained for approximately 24 hours (second time).

In the depressurization/drying step SB21, when the internal space of the chamber 11 is depressurized, hydrogen peroxide remaining in the object is efficiently evaporated, so that hydrogen peroxide is removed from the object. Note, however, that when a certain period of time elapses from the start time of the depressurization/drying step SB21, the evaporation amount of hydrogen peroxide decreases. Therefore, in the present embodiment, the control device 61 ends the depressurization/drying step SB21 when approximately 24 hours have elapsed from the start time of the depressurization/drying step SB21. Note that as a cause of the decrease in the evaporation amount of hydrogen peroxide, the concentration of hydrogen peroxide remaining in the object is high, or it is difficult to evaporate due to bonding of hydrogen peroxide to the object.

After completion of the depressurization/drying step SB21, the atmospheric air supply step SB22 is performed. In the atmospheric air supply step SB22, the control device 61 controls a repressurization unit 50 so that the atmospheric air is supplied to the internal space of the chamber 11. As described above, the repressurization unit 50 has a filter 51 such as a HEPA filter. Clean air (atmospheric air) that has passed through the filter 51 is supplied to the internal space of the chamber 11. When air (atmospheric air) is supplied to the internal space of the chamber 11, the pressure in the internal space of the chamber 11 increases. The control device 61 may increase the pressure in the internal space of the chamber 11 to the atmospheric pressure or to a pressure lower than the atmospheric pressure.

In the present embodiment, in the atmospheric air supply step SB22, the pressure in the internal space of the chamber 11 increases to a pressure (approximately 90000 Pa) lower than the atmospheric pressure, so that hydrogen peroxide remaining in the object is discharged to the external space of the chamber 11 together with the atmospheric air.

After the atmospheric air supply step SB22 is completed, the depressurization step SB23 of depressurizing the internal space of the chamber 11 is performed.

After the depressurization step SB23 is completed, the water vapor supply step SB24 of supplying water vapor to the internal space of the chamber 11 is performed. In the water vapor supply step SB24, when water vapor is supplied to the internal space of the chamber 11, hydrogen peroxide remaining in the object dissolves into the water vapor. Hydrogen peroxide is peeled off from the object by the water vapor. As a result, hydrogen peroxide is removed from the object.

After the water vapor supply step SB24 is completed, the atmospheric air supply step SB25 is performed. In the atmospheric air supply step SB25, the control device 61 controls the repressurization unit 50 so that the atmospheric air is supplied to the internal space of the chamber 11.

A pulse step including the depressurization step SB23, the water vapor supply step SB24, and the atmospheric air supply step SB25 is performed a plurality of times. The number of pulses indicating the number of times of performing the pulse step is a predetermined value. Note that the user may operate an input device 62 to input the number of pulses to the control device 61. The number of pulses may be arbitrarily determined by the user. The control device 61 determines whether or not the number of times the pulse step has been performed has reached the number of pulses (step SB26).

In step SB26, if it is determined that the number of pulses has not been reached (step SB26: No), the control device 61 repeats the processing from step SB23 to step SB25 until the number of pulse steps reaches the number of pulses.

In step SB26, if it is determined that the number of pulses has been reached (step SB26: Yes), the control device 61 ends the removal processing SB.

### REFERENCE SIGNS LIST

- 10: Chamber unit
- 11: Chamber
- 12: Door
- 13: Heating unit
- 14: Pressure sensor
- 15: Temperature sensor
- 16: Injection line
- 17: Injection line
- 18: Injection line
- 19: Injection line
- 20: Hydrogen peroxide supply unit
- 21: Bottle
- 22: Extraction line
- 23: Tube pump
- 24: Reservoir
- 25: Filter
- 26: Evaporator
- 27: Supply line
- 28: Valve
- 29: Heater
- 30: Vapor cleaning unit
- 31: Bottle
- 32: Extraction line
- 33: Tube pump
- 34: Reservoir
- 35: Filter
- 36: Evaporator
- 37: Supply line
- 38: Valve
- 39: Heater
- 40: Depressurization unit
- 41: Vacuum pump
- 42: First catalyst tank
- 43: Second catalyst tank
- 44: First heater
- 45: Second heater
- 46: Exhaust line
- 50: Repressurization unit
- 51: Filter
- 52: Introduction line
- 60: Control unit
- 61: Control device
- 62: Input device
- 71: Valve
- 72: Valve
- 73: Valve
- 74: Valve
- 77: Valve
- 78: Valve
- 100: Sterilization device
- SA: Sterilization processing
- SB: Removal processing
- SB11: Depressurization/drying step
- SB12: Water vapor supply step
- SB13: Atmospheric air supply step
- SB14: Atmospheric air retention step
- SB15: Depressurization step
- SB16: Water vapor supply step
- SB17: Atmospheric air supply step
- SB18: Atmospheric air retention step

## Claims

1. A method for removing hydrogen peroxide remaining in an object, the method comprising:
a depressurization step of depressurizing an internal space of a chamber accommodating the object;
a water vapor supply step of supplying water vapor to the internal space in a state where the internal space is depressurized; and
an atmospheric air supply step of supplying clean atmospheric air passing through a HEPA filter to the internal space after water vapor is supplied to the internal space.

2. The method for removing hydrogen peroxide according to claim 1, wherein a pulse step including the depressurization step, the water vapor supply step, and the atmospheric air supply step is performed a plurality of times.

3. The method for removing hydrogen peroxide according to claim 2, wherein
the internal space is brought to a first pressure by the depressurization step, and
a depressurization/drying step of depressurizing the internal space to a second pressure lower than the first pressure is performed before the pulse step.

4. The method for removing hydrogen peroxide according to claim 3, wherein
a state where the internal space is at the first pressure is maintained for a first time in the depressurization step, and
a state where the internal space is at the second pressure is maintained for a second time longer than the first time in the depressurization/drying step.

5. The method for removing hydrogen peroxide according to claim 3, wherein a sterilization step of sterilizing the object with the hydrogen peroxide in the internal space is performed before the depressurization/drying step.
